# EUROPEAN PATENT APPLICATION

(11) **EP 4 768 570 A1**
(43) Date of publication of application: **01.07.2026**
(21) Application number: 23949352.1
(22) Date of filing: 21.08.2023
(51) Int. Cl.: C12N 5/071, C12N 5/10, C12N 15/85, A61K 35/407, A61P 1/16

(54) **LIVER PRECURSOR CELL AND CONSTRUCTION METHOD THEREFOR AND USE THEREOF**

(71) Applicant: Shanghai Celliver Biotechnology Co., Ltd., Shanghai 201210 (CN)
(72) Inventor: ZHOU, Shen ao, Shanghai 201210 (CN); ZHANG, Qin, Shanghai 201210 (CN); ZHOU, Li, Shanghai 201210 (CN)
(74) Representative: Bayramoglu et al.
(86) International application number: PCT/CN2023/114029
(87) International publication number: WO 2025/039158

(57) **Abstract**

The present invention application is related to a hepatic precursor cell and a method and an application for establishing a hepatic precursor cell line. The method for establishing a hepatic precursor cell line comprises: S0: preparing hepatic precursor cells, miscible liquids, and a reprogramming culture medium, wherein the miscible liquids comprises a serum-free medium, polyethyleneimine, and a CLP plasmid; S1: transfecting the hepatic precursor cells with the miscible liquids to obtain transduced hepatic precursor cells; and S2: passing the transduced hepatic precursor cells into the reprogramming culture medium for subculture to obtain immortalized hepatic precursor cells. The present invention application resolves the prior art limitations of immortalization genes, which cannot transform hepatic precursor cells into immortalized cell lines or enable sustained in vitro subculturing.

## Description

### TECHNICAL FIELD

the present invention application is related to the field of biotechnology, particularly to hepatic precursor cells and methods and applications for establishing a system threrof.

### BACKGROUND TECHNOLOGY

Cell therapy represents a relatively safe, effective, and promising treatment modality. Primary hepatocytes are the earliest cell source clinically applied in the treatment of liver diseases. Between 1999 and 2006, in eight clinical cases of primary hepatocyte transplantation for Crigler-Najjar Syndrome (abbreviated as CNS), all patients exhibited an overall reduction of over 50% in serum bilirubin levels. In 2019, the team led by A. Dhawan in the UK employed microencapsulated primary hepatocytes for intraperitoneal transplantation in eight children with acute liver failure, among whom four avoided liver transplantation and three successfully bridged to transplantation during the acute phase. However, hepatocyte-based cell therapy and disease modeling have been constrained by the limitations of primary hepatocyte expansion in vitro.

Cell immortalization refers to the process in which cells, during in vitro culture, acquire the ability to proliferate indefinitely by avoiding the normal aging and death processes due to intrinsic genetic alterations or various external stimuli such as viral infections. Consequently, these cells can be cultured for extended periods and undergo unlimited division in vitro. Immortalizing primary hepatocytes in vitro may represent a potential solution to address the quantitative limitations of primary hepatocyte culture in vitro.

Currently, there are three main categories of methods for immortalizing primary hepatocytes:
The first category involves immortalization of primary hepatocytes using viral genes, such as simian virus 40T antigen (SV40T), adenovirus EIA and EIB genes, human papillomavirus E6/E7 genes, and Epstein-Barr virus (EBV) immortalization. These methods can inactivate cell cycle regulatory proteins pRB and p53 through various mechanisms, enabling cells to bypass the senescence phase and undergo continuous subculturing. Kobayashi et al. transfected 21-week human fetal liver cells with a plasmid containing the SV40T tumor antigen (PSV3neo), resulting in the immortalized hepatocyte line OUMS-29. Immortalization methods for different viruses exhibit adaptive differences for various cell types. The E6/E7 genes of HPV16 can efficiently establish immortalized cell lines, and extensive studies have demonstrated that HPV16-eE/E7 is highly suitable for hepatocyte immortalization. Professor Yan Hexin's team utilized HPV16-E6/E7 genes to immortalize hepatic precursor cells(or liver precursor cells), which can be subcultured in vitro for over 40 generations and express surface markers associated with hepatic precursor cells.
The second category involves telomerase and its catalytic subunit-dependent hepatocyte immortalization. As the catalytic subunit of telomerase, hTERT binds to related proteins to activate telomerase, thereby extending the survival cycle of cells in vitro, serving as a positive regulator of telomerase. Wege et al. reported that transfection of hTERT gene into human fetal liver cells resulted in telomere elongation, enabling the cells to surpass the replication senescence phase. These cells could be passaged over 300 generations in vitro while maintaining liver-specific functions.
The third category is restorable immortalization. Kobayashi et al. from Japan introduced a recombinant SSR69 retrovirus, which carried the SV40T antigen, herpes simplex virus thymi •dine kinase (HSV-TK), and streptothrix pyogenes (Streptomyces pyogenes) resistance genes, with loxP sites flanking both ends, into primary adult hepatocytes. This successfully established the restorable immortalized hepatocyte line NKTK-3. By transfecting the hepatocytes with a recombinant adenovirus vector expressing Cre recombinase, the SV40T gene was effectively excised, and NKTK-3 cells no longer expressed SV40T, restoring the hepatocyte line to its pre-immortalized state.

The current techniques for the immortalization of hepatic precursor cells are not mature. Most immortalization genes cannot transform hepatic precursor cells into immortalized cell lines, nor can they support continuous subculturing in vitro.

Therefore, it is necessary to provide a hepatic precursor cell and methods and applications for establishing a cell line thereof to solve the above problems in the existing technology.

### CONTENT OF THE INVENTION

The technical problem to be solved by the present invention application is to provide a hepatic precursor cell and methods and applications for establishing a cell line thereof, to solve the problems that immortalized genes in existing technologies cannot transform hepatic precursor cells into immortalized cell lines, nor enable continuous subculturing in vitro.

In accordance with the present invention application, to solve the above technical problems, there is provided a method for establishing a hepatic precursor cell line, comprising the following steps:
S0: providing hepatic precursor cells, miscible liquids, and a reprogramming culture medium, wherein the miscible liquids comprises a serum-free medium, polyethyleneimine, and a CLP plasmid;
S1: transfecting the hepatic precursor cells with the miscible liquids to obtain transduced hepatic precursor cells; and
S2: subculturing the transduced hepatic precursor cells in the reprogramming culture medium to obtain immortalized hepatic precursor cells.

The method for establishing a hepatic precursor cell line according to the present invention application has the following advantages:
The hepatic precursor cells are first transfected with the miscible liquids to obtain transduced cells, which are then subcultured in a reprogramming culture medium to generate immortalized cells. The use of CLP plasmid for transfection ensures non-toxicity, and the reprogramming culture medium enables continuous subculturing in vitro. Therefore , the method for establishing a hepatic precursor cell line according to the present invention application solves the limitations of existing immortalization techniques, which fail to transform hepatic precursor cells into immortalized cell lines or support sustained subculturing in vitro.

Preferably, the volume ratio of the serum-free medium to the polyethyleneimine is (500-1000):1.

Preferably, a final concentration of the CLP plasmid is (1-5) µg/ µl.

Preferably, the step of providing hepatic precursor cells comprises: culturing hepatocytes in the reprogramming culture medium for expansion, followed by replacing the reprogramming culture medium with a serum-depleted medium to continue the culture and obtain hepatic precursor cells.

Preferably, the reprogramming culture medium comprises DMEM/F12 basal medium and the following components in concentrations relative to DMEM/F12 basal medium: 15-25 ng/mL of hepatocyte growth factor (HGF), 15-25 ng/mL of epidermal growth factor (EGF), 5-15 µM of ROCK kinase inhibitor Y27632,1-5 µM of Wnt signaling agonist ChIR99021, 1-5 µ M of TGF- β signaling inhibitor A8301, 1-5 µ M of sphingosine-1-phosphate(S1P), and 5 µM of indole-3-acetic acid (LPA).

Preferably, the reprogramming culture medium further comprises N2 nutritional supplement, B27 nutritional supplement, sodium pyruvate, and ascorbic acid.

Preferably, the reprogramming culture medium comprises DMEM/F12 basal medium and the following components in concentrations relative to DMEM/F12 basal medium: 1-10% N2 nutrient supplement, 1-10% B27 nutrient supplement, 1 mM sodium pyruvate, 5-15 µg/mL ascorbic acid, 15-25 ng/mL hepatocyte growth factor (HGF), 15-25 ng/mL epidermal growth factor (EGF), 5-15 µM ROCK kinase inhibitor Y27632, 1-5 µM WNT signaling agonist CHIR99021, 1-5 µM TGF- β signaling inhibitor A8301, 1-5 µM sphingosine-1-phosphate (S1P), and 5 µM indole-3-acetic acid (LPA).

In accordance with the present invention application, to solve the above technical problems, there is also provided a hepatic precursor cell, which is prepared by the method for establishing the hepatic precursor cell line.

The hepatic precursor cell according to the present invention application has the following advantages:
By employing CLP plasmid to transfect the hepatocyte precursor cells without inducing cytotoxicity, followed by subculturing in a reprogramming medium, the present invention application enable the transduced hepatocyte precursor cells to undergo continuous subculturing in vitro. Therefore, the present invention application addresses the issue in prior art where immortalized genes cannot transform hepatic progenitor cells into immortalized cell lines, nor enable sustained subculturing in vitro.

Preferably, the hepatic precursor cells negatively express CD34, CD45, and HLA-DRPQ, while the hepatic precursor cells positively express CD24 and CD326.

In accordance with the present invention application, to solve the above technical problems, there is further provided an application of hepatic precursor cells, wherein the hepatic precursor cells are used in the preparation of a drug for treating acute liver failure.

Compared with the prior art, the application of the hepatic precursor cells according to the present invention application has the beneficial effects in that: the injection containing hepatic precursor cells exhibits significant therapeutic efficacy against the acute liver failure model induced by S-gal inducer combined with LPS inducer in mice.

### DESCRIPTION OF THE DRAWINGS

Fig. 1 illustrates a schematic diagram of a plasmid in an embodiment of the present invention application.
Fig. 2 illustrates the morphological characteristics of cell line B42 in an embodiment of the present invention application.
Fig. 3 illustrates the morphological characteristics of cell line B42 which is the fifteenth generation cell line in an embodiment of the present invention application.
Fig. 4 illustrates the flow cytometry detection results of cell line b42 which negatively expresses CD34 in an embodiment of the present invention application.
Fig. 5 illustrates the flow cytometry detection results of cell line b42 which negatively expresses CD45 in an embodiment of the present invention application.
Fig. 6 illustrates the flow cytometry detection results of cell line b42 which negatively expresses HLA-DRPQ in an embodiment of the present invention application.
Fig. 7 illustrates the flow cytometry detection results of the cell line b42 which positively expresses CD24 in an embodiment of the present invention application.
Fig. 8 illustrates the flow cytometry detection results of the cell line b42 which positively expresses CD326 in an embodiment of the present invention application.
Fig. 9 is a comparative schematic diagram of the survival rates in the experimental group and control group of acute liver failure models in mice according to an embodiment of the present invention application.

### SPECIFIC IMPLEMENTATIONS

To clarify the objectives, technical solutions, and advantages of the embodiments of the present invention application, the technical solutions are described in detail below. Note that the described embodiments represent only a subset of the present invention application, not its entirety. Any additional embodiments developed by skilled persons in the art without inventive effort are protected under the present invention application. Unless otherwise specified, technical or scientific terms herein shall be interpreted as commonly understood by those skilled in the art. The term 'include' and similar expressions herein denote that the elements or objects preceding such terms encompass those listed and their equivalents, without excluding other elements or objects.

Virus transfection, adenovirus transfection and electroporation transfection are the common methods to construct immortalized cell lines.

Viruses can introduce exogenous genes into cells, thereby enabling genome modification. Stable immortalized cell lines can be constructed for long-term cell culture and experimental research by transfecting virals. Viral transfection efficiently delivers exogenous genes into cells, enabling target gene expression. However, lentiviral infection requires substantial lentiviral vectors and reagents, resulting in high costs. Additionally, strict laboratory controls are necessary to prevent safety issues such as viral leakage. Lentiviral infection is limited to specific cell types, and different cell types exhibit varying infection efficiencies with lentiviruses, thereby restricting the applicability of lentiviral infection for constructing immortalized cell lines. Lentiviral infection may introduce foreign genes, leading to cellular mutations or even carcinogenesis. Furthermore, lentiviruses themselves may pose safety risks, such as inducing cell death or inflammatory responses following viral infection.

Adenovirus infection is a non-pathogenic virus capable of introducing exogenous genes into cells, thereby enabling genomic modification. Through adenovirus infection, stable immortalized cell lines can be constructed for long-term cell culture and experimental research. However, adenovirus infection has the following limitations: it is only applicable to specific cell types, and the infection efficiency varies among different cell types. Consequently, the applicability of adenovirus infection for constructing immortalized cell lines is restricted. Additionally, adenovirus infection requires the use of large quantities of adenoviral vectors and reagents, resulting in high costs. Furthermore, adenovirus infection necessitates strict control of the laboratory environment to prevent safety issues such as viral leakage.

Electroporation transfection utilizes high-voltage electrical pulses to introduce exogenous DNA into cells, thereby enabling genomic modification, and this technique facilitates the establishment of stable immortalized cell lines for long-term cell culture and experimental research. However, the method requires high-voltage pulses, which may pose safety risks to operators if improperly performed. Additionally, electroporation transfection can cause cellular damage and death, potentially compromising experimental outcomes. Different cell types exhibit varying adaptability to this technique, with some cells being damaged or killed during the process. Consequently, the applicability of electroporation transfection for constructing immortalized cell lines is limited. Furthermore, cell lines established through this method may lose genomic stability due to instability, leading to inconsistent gene expression and affecting experimental results.

The current techniques for constructing hepatocyte precursors are not mature enough to achieve in vitro continuous passage.

The present invention application employs plasmid transfection to directly immortalize cells, thereby obtaining hepatic precursor cells (HepLPCs) with clear donor origins and traceable information. The plasmid CLP-ET, which carries the exogenous gene HPV-16-E7-delta TK, is transduced into the HepLPC genome using PEI transfection reagent, establishing a stably transfected cell line. The PEI transfection reagent, a type of cationic polymer transfection reagent, binds to DNA to form complex particles that enter cells via endocytosis. After DNA enters the cells, the exogenous gene can be randomly integrated into the cellular genomic DNA. The transfection reagent does not introduce viral origins or nucleic acid sequences such as transposases into the inserted gene fragment, ensuring greater safety for subsequent clinical use of the cell line.

After cell transfection, stable transfected cell lines were selected through two rounds of monoclonal screening. The functionality of the selected cell lines was validated through experiments including surface marker detection, cell function verification, pharmacodynamic pre-experiments, genome sequencing, and metabolomics sequencing. The cell lines resolved the issue of immortalization of hepatic progenitor cells and posed no safety concerns as they were modified using only plasmids.

In accordance with the present invention application, there is provided a method for establishing a hepatic precursor cell line, comprising the following steps:
S0: preparing hepatic precursor cells, miscible liquids, and a reprogramming medium, wherein the miscible liquids comprises a serum-free medium, polyethyleneimine, and CLP plasmids;
S1: transfecting the hepatic precursor cells with the miscible liquids to obtain transduced cells; and
S2: subculturing the transduced cells in the reprogramming culture medium to generate immortalized hepatic precursor cells.

Specifically, the present invention application involves transfecting hepatic precursor cells with a mixed solution to obtain transduced cells, which are then cultured in a reprogramming culture medium for subculturing to generate Eternalized hepatoblasts. The CLP plasmid is used for transfection without inducing cytotoxicity, and subculturing in the reprogramming culture medium enables continuous in vitro passage of the transduced cells. Thus, the present invention application resolves the limitations of existing immortalization techniques, which fail to transform hepatic precursor cells into immortalized cell lines or support sustained in vitro passage.

In some embodiments of the present invention application, the volume ratio of the serum-free medium to the polyethyleneimine is (500-1000):1.

In some embodiments of the present invention application, the final concentration of the CLP plasmid is (1-5) µg/µl.

In some embodiments of the present invention application, the step of preparing hepatic precursor cells comprises: culturing hepatocytes in the reprogramming culture medium for expansion, followed by replacing the reprogramming culture medium with a serum-reduced medium to continue the culture and obtain hepatic precursor cells.

In some embodiments of the present invention application, the reprogramming culture medium comprises DMEM/F12 basal medium and, in terms of the content relative to the DMEM/F12 basal medium: 15-25 ng/mL of hepatocyte growth factor (HGF), 15-25 ng/mL of epithelial growth factor (EGF), 5-15 µM of ROCK kinase inhibitor Y27632, 1-5 µM of Wnt signaling pathway agonist Chir99021, 1-5 µM of TGF-β signaling inhibitor A8301,1-5 µM of sphingosine-1-phosphate (S1P), and 5 µM of indole-3-acetic acid (LPA).

In some embodiments of the present invention application, the reprogramming culture medium further comprises N2 nutritional supplement, B27 nutritional supplement, sodium pyruvate, and ascorbic acid.

In some embodiments of the present invention application, the reprogramming culture medium comprises DMEM/F12 basal medium and, in terms of the content relative to the DMEM/F12 basal medium: 1-10% N2 nutrient supplement, 1-10% B27 nutrient supplement, 1 mM sodium pyruvate, 5-15 µg/mL ascorbic acid, 15-25 ng/mL hepatocyte growth factor (HGF), 15-25 ng/mL epidermal growth factor (EGF), 5-15 µM ROCK kinase inhibitor Y27632,1-5 µM WNT signaling agonist Chir99021,1-5 µM TGF-β signaling inhibitor A8301,1-5 µM S1P (sphingosine-1-phosphate), and 5 µM indole-3-acetic acid (LPA).

In accordance with the present invention application, there is also provided a hepatic precursor cell, which is prepared by the method for establishing the hepatic precursor cell line.

Specifically, the present invention application employs CLP plasmids to transfect hepatic precursor cells without inducing cytotoxicity. The transduced cells are then passaged in a reprogramming culture medium, enabling sustained in vitro propagation. The method resolves the limitations of prior art, where immortalization genes fail to transform hepatic precursor cells into immortalized cell lines and cannot support continuous in vitro passage.

In some embodiments of the present invention application, the hepatic precursor cells negatively express CD34, CD45, and HLA-DRPQ, while the hepatic precursor cells positively express CD24 and CD326.

In accordance with the present invention application, there is further provided an application of hepatic precursor cells, wherein the hepatic precursor cells are used in the preparation of a drug for treating acute liver failure.

Specifically, the injection containing hepatic precursor cells demonstrated significant efficacy in the acute liver failure model induced by D-gal and LPS.

### The Embodiments

### I. Plasmid transfection of hepatic progenitor cells

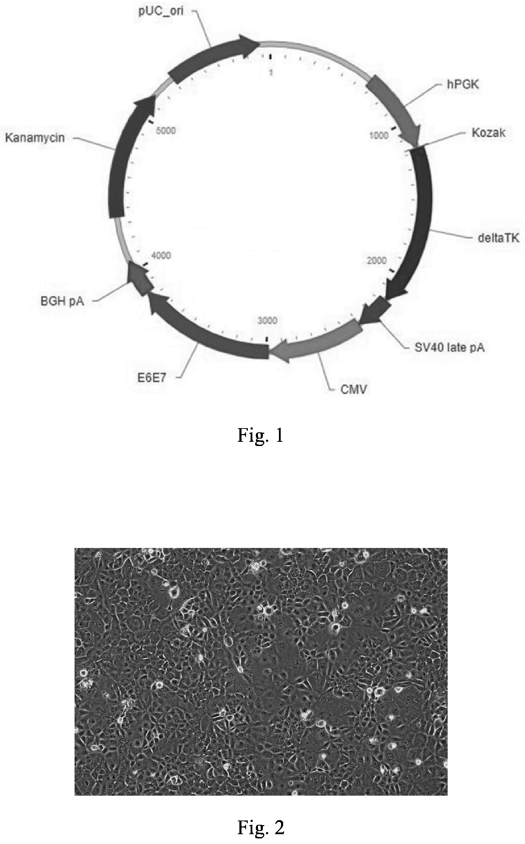

Fig. 1 illustrates a schematic diagram of the plasmid in an embodiment of the present invention application.

The insertion sites of E6E7 are provided by the inventors and synthesized by Yunzhou Biotechnology (Guangzhou) Co., Ltd. The resulting E6E7 is ligated into the vector via enzymatic digestion, and the constructed CLP plasmid is shown in Fig. 1.

The composition of the reprogramming culture medium includes: DMEM/F12 basal medium, supplemented with: 1% N2 nutrient supplement (100X), 1% B27 nutrient supplement (50X), 1 mM sodium pyruvate, 10 µg/mL ascorbic acid, 20 ng/mL hepatocyte growth factor (HGF), 20 ng/mL epidermal growth factor (EGF), 10 µM ROCK kinase inhibitor Y27632,3 µM WNT signaling agonist CHIR99021,1 µM TGF- β signaling inhibitor A8301,1 µM S1P (sphingosine-1-phosphate), and 5 µM indole-3-acetic acid (LPA). The DMEM/F12, N2 nutrient supplement, B27 nutrient supplement, and sodium pyruvate are sourced from Invitrogen; ascorbic acid from Sigma-Aldrich; HGF and EGF from Novoprotein; while Y27632, CHIR99021, A8301, S1P, and LPA are sourced from TargetMol.

Unless otherwise specified, the medium used for subculture in the embodiments of the present invention application is the reprogramming culture medium.

Two hepatic precursor cell lines were resuscitated, with 200,000 cells per well inoculated into a 6-well plate. The cells were cultured in 2 mL of reprogramming culture medium overnight until adhesion was achieved, followed by transfection. Thirty minutes prior to transfection, the reprogramming culture medium was replaced with 1 mL of serum-reduced medium, and the cells were returned to incubation in a culture incubator to obtain the transfection-ready hepatic precursor cells.

Take two 1.5 mL centrifuge tubes labeled as the first and second tubes. Add 50 µL of serum-free medium and 6 µL of polyethyleneimine to the first tube, then mix rapidly with a pipette for 10 cycles to obtain the first mixture. Add 50 µL of serum-free medium and 2 µ g of CLP plasmid to the second tube, mix with a pipette, and let it stand for 10 minutes to obtain the second mixture. Transfer the second mixture from the second tube to the first tube using a pipette, mix rapidly for 10 cycles to obtain the final mixture. Add the miscible liquids dropwise to a 6-well plate containing pre-transfected hepatic precursor cells, then transfer the plate to a CO2 incubator for transfection. After 8 hours of transfection, replace the serum-free medium in the plate with 2 mL of reprogramming culture medium for subculturing.

Recovery of primary hepatic progenitor cells from Pr generation and inoculation into 6-well platesReprogrammed primary hepatic precursor cells were inoculated into 6-well plates for CLP-ET plasmid transfection and maintained in reprogramming culture medium. When the cell fusion rate exceeded 80%, subculturing was initiated. Each subculture of transfected cells was designated as P0+1, all cultured in 10cm cell culture dishes. When the cell fusion rate surpassed 80% at P0+5 subculture, the cells were seeded into 96-well plates using the limited dilution method.

When the fusion rate of PO+5 generation primary hepatic precursor cells reached 80%, they were passaged for culture and subjected to monoclonal selection, the cell suspension was diluted using the limited dilution method to a concentration of 70 cells/well/20 µL, a total of 200 µL of culture medium was added to each well in a 96-well plate, equivalent to approximately 0.7 cells/well/200 µL, four 96-well plates were used for the experiment, the clone numbers were assigned in the order of the selected monoclonal cells, and a total of 21 monoclonal cell lines were isolated and designated as P1 generation monoclonal cells.

The P1 generation monoclonal cells were cultured in a 96-well plate after 3 days of plate seeding, individual wells were selected. When the confluence of P1 generation monoclonal cells reached over 80%, subculturing was performed. Following routine digestion and subculturing, all P1 generation monoclonal cells in the 96-well plate were digested. After centrifugation, the P1 generation monoclonal cells were directly inoculated into a 24-well plate and cultured in a CO₂ incubator to obtain P1+1 generation monoclonal cells. The culture medium in the 24-well plate was replaced every 2-3 days.

The P1+1-generation monoclonal cells were cultured in a 24-well plate. When the cell confluence in the P1+1-generation monoclonal cells reached over 80%, the cells were subcultured. After routine digestion and passage, all P1+1-generation monoclonal cells in the 24-well plate were digested, centrifuged, and directly inoculated into a 6-well plate. The 6-well plate was then incubated in a CO2 incubator to obtain P1+2-generation monoclonal cells. The culture medium in the 6-well plate was replaced every 2-3 days.

The P1+2-generation monoclonal cells were cultured in a 6-well plate. When the cell confluence in the P1+2-generation monoclonal cells reached over 80%, the cells were subcultured. The culture supernatant was collected and cryopreserved at-20 ° C for detection of AAT secretion and urea synthesis. All P1+2-generation monoclonal cells in the 6-well plate were digested, centrifuged, and counted. The cells were then inoculated at a density of 10,000 cells/cm² into 10 cm culture dishes and incubated in a CO₂incubator to obtain P1+3-generation monoclonal cells. The culture medium in the 10 cm dishes was replaced every 2-3 days.

The P1+3-generation monoclonal cells were cultured in 10 cm dishes. When the cell confluence in the P1+3-generation monoclonal cells reached over 80%, subculturing was performed. After routine digestion and passage, all P1+3-generation monoclonal cells in the 10 cm dishes were digested, centrifuged, and counted. The cells were then inoculated at a density of 10,000 cells/cm² into 10 cm dishes and cultured in a COzincubator to obtain p14-generation monoclonal cells. Upon full growth, the p14-generation monoclonal cells were harvested and cryopreserved. The P1+3-generation monoclonal cells in the 10 cm dishes were replaced with new medium every 2-3 days.

After multiple passages, 11 out of 21 monoclonal cells remained viable. The 11 cells were labeled as A, B, C, D, E, F, G, H, I, J, K, L, M, and N, respectively. The AAT, ALB, and urea levels of the 11 monoclonal cells were measured, with the results presented in Table 1.

**Table 1**

| name | ALB secretion (µg/million cell/24h) | AAT secretion (ng/million cell/24h) | Urea synthesis (ng/million cell/24h) |
|---|---|---|---|
| A | 231.57 | 231.57 | 179.57 |
| B | 212.98 | 222.98 | 196.98 |
| C | 180.47 | 165.47 | 126.47 |
| D | 140.86 | 140.86 | 140.86 |
| E | 120.75 | 120.75 | 120.75 |
| F | 261.98 | 162.58 | 162.58 |
| G | 157.25 | 157.25 | 151.25 |
| H | 149.96 | 149.96 | 145.96 |
| I | 80.57 | 80.57 | 80.57 |
| J | 95.76 | 95.76 | 95.76 |
| K | 107.76 | 107.76 | 107.76 |
| L | 112.7 | 112.7 | 112.7 |
| M | 40.67 | 40.67 | 40.67 |
| N | 75.46 | 75.46 | 75.46 |

Based on the synthesis detection results of AAT, ALB, and urea in Table 1, 6 monoclonal cells were selected from 11 P1+2-generation monoclonal cells for the second round of screening. The 6 monoclonal cells were designated as 6 P2-generation monoclonal cells, labeled as A, B, C, F, G, and H, respectively.

The six P2 monoclonal cell lines were resuspended in reprogramming culture medium and seeded on plates. When the confluence of the six P2 monoclonal cell lines reached 80%, they were passaged for further cultivation. Subsequently, the monoclonal cells were selected and diluted using the finite dilution method to a density of 70 cells/20 µL. A total of 200 µL of culture medium which equates to approximately 0.7 cells/well/200 µL was added to each well of the 96-well plate.

After 24 hours of plate incubation, select wells containing only single P2- generation monoclonal cells in the 96-well plate and label them. When the confluence of P2 generation monoclonal cells reaches 80%, a subculture, a routine digestion and a subculture were performed. After digestion of all P2 generation monoclonal cells in the 96-well plate was completed and centrifuged, the P2 generation monoclonal cells were directly inoculated into a 24-well plate. The 24-well plate was placed in a CO2 incubator for cultivation to obtain P2+1 generation monoclonal cells. The P2+1 generation monoclonal cells in the 24-well plate should be replaced with new medium or subcultured approximately every 3-4 days.

When the confluence of P2+1-generation monoclonal cells in the 24-well plate reaches over 80%, subculturing is performed, in which the cells are routinely digested and transferred, and all P2+1-generation monoclonal cells in the 24-well plate are digested, centrifuged, and directly inoculated into a 6-well plate. The 6-well plate is then incubated in a CO₂ incubator to obtain P2+2-generation monoclonal cells. The P2+2-generation monoclonal cells in the 6-well plate are replaced with new medium or transferred every 3-4 days.

When the confluence of P2+2-generation monoclonal cells in the 6-well plate reaches over 80%, subculturing is performed, in which the cells are routinely digested and resuspended, and all P2+2-generation monoclonal cells in the 6-well plate are digested, centrifuged, and counted. The cells are then seeded at a density of 20,000 cells/cm² into a 10 cm culture dish and incubated in a CO₂incubator to obtain P2+3-generation monoclonal cells. The supernatant of P2+3-generation monoclonal cells is collected and centrifuged at 200 g for 5 minutes to remove debris. The cells are stored at-80 ° C for detection of urea and AAT synthesis.

When the confluence of P2+3-generation monoclonal cells in a 10 cm culture dish reaches over 80%, subculturing is performed, in which Routine digestion and subculturing are conducted. All P2+3-generation monoclonal cells in the 10 cm culture dish are digested, centrifuged, and counted. The cells are then inoculated into T75 flasks at a density of 20,000 cells/cm² and cultured in a CO₂incubator to obtain P2+4-generation monoclonal cells.

**Table 2 Test Results**

| Name | AAT secretion (ng/million cell/24h) | Urea synthesis (ng/million cell/24h) |
|---|---|---|
| A14 | 270.57 | 179.57 |
| B42 | 350.98 | 220.98 |
| C17 | 101.47 | 116.47 |
| F25 | 140.58 | 132.58 |
| G47 | 125.25 | 151.25 |
| H53 | 149.96 | 145.96 |

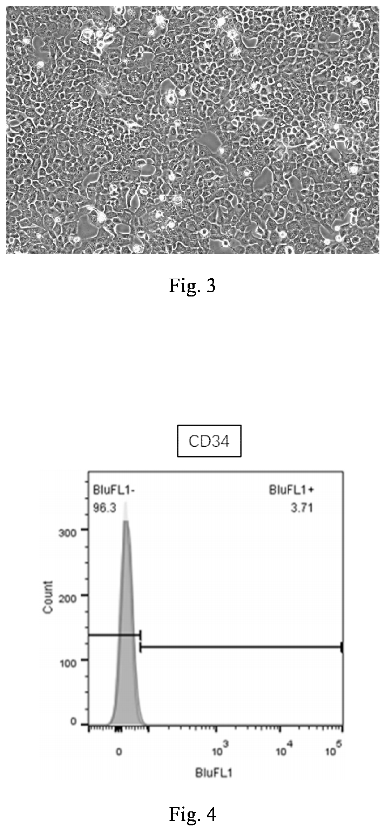

Fig. 2 illustrates the morphological characteristics of cell line b42 in an embodiment of the present invention application. Fig. 3 illustrates the morphological characteristics of cell line b42 cultured up to the fifteenth generation in an embodiment of the present invention application.

By screening, cell line b42 was identified as a immortalized hepatic precursor cell. The cell line b42 was cultured through subculturing to the fifteenth generation, yielding the fifteenth-generation cell line b42. Morphological images of cell line b42 and the fifteenth-generation cell line b42 were photographed, resulting in Figs. 2 and 3. As shown in Figs. 2 and 3, the morphology of the fifteenth-generation cell line b42 is nearly identical to that of cell line b42, indicating that cell line b42 retains the characteristics of hepatic precursor cells even after subculturing which confirms the immortalization of cell line b42.

Flow cytometry was performed to identify cell line b42. The specific steps include:
Surface marker staining of cell line B42: collect the cultured cell line B42, aspirate the medium, and rinse with 5 ml sterile PBS buffer. Then, 2 ml of trypsin digestion solution was added to the dish for digestion to obtain a cell mixture. The cell mixture was transferred to a 15ml centrifuge tube, place the tube in a centrifuge, and centrifuge at 200g for 5 minutes was completed. The supernatant of the centrifuged cell mixture was discarded to obtain the cell pellet.

100 µl of staining buffer was added to the cell pellet to resuspend it. The resuspended cell pellet was transferred to 61.5ml centrifuge tubes, with 100 µ1 per tube. 5 µl of the target flow cytometry antibody was added to each of the 61.5ml centrifuge tubes, and the miscible liquids was stirred. The tubes were then placed in a 2-8° C refrigerator for 30 minutes. Sterile PBS buffer was added to each tube at a concentration of 800 µ1 per tube, and the tubes were placed in a centrifuge. The miscible liquids was centrifuged at 300g for 5 minutes. After centrifugation, the supernatant was discarded and the centrifuge tubes on a tube rack was incubated at 37° C for 20 minutes. 400 µl of staining buffer was added to each centrifuge tube after incubation to resuspend the cell pellet, then the esuspended cell mixture was transferred to a flow cytometer tube for surface marker flow cytometric detection.

The antibodies used for surface marker flow cytometric detection are: CD24, CD326, CD34, CD45, and HLA-DR/DP/DQ. Specifically, CD34 is purchased from abcam (Art No.: ab81289); CD45 is purchased from Abcam (Art No: ab40763); HLA-DR/DP/DQ is purchased from Abcam (Art No: ab7856); CD326 is purchased from BD Biosciences (Art No: 565399); CD24 is purchased from abcam (Art No: ab290730); the staining buffer is purchased from Biosharp (Art No: Bl1136A); and the trypsin digestion solution is purchased from Yuanpei (Art No.: S310JV).

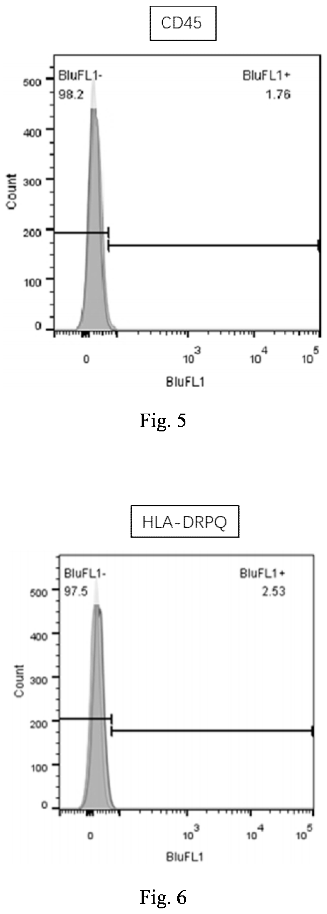

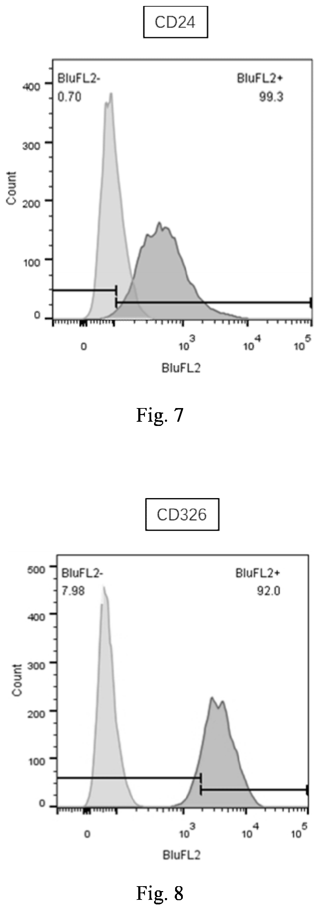

Fig. 4 illustrates the flow cytometry results of cell line B42 negative for CD34 expression; Fig. 5 illustrates the results for cell line B42 negative for CD45 expression; Fig. 6 illustrates the results for cell line B42 negative for HLA-DRPQ expression; Fig. 7 illustrates the results for cell line B42 positive for CD24 expression; and Fig. 8 illustrates the results for cell line B42 positive for CD326 expression.

Flow cytometry analysis of cell line B42 revealed negative expression of CD34, CD45, and HLA-DRPQ, and positive expression of CD24 and CD326, as shown in Figs. 4, 5, 6, 7, and 8.

### II. Therapeutic Effects of Cell Line b42 on Liver Failure

1) Preparation of d-gal inducer: Weigh 500 mg d-gal, add 10ml normal saline, and vortex to dissolve to obtain D-gal inducer with a concentration of 50 mg/ml. Store the d-gal inducer at 2-8° C and use it within 2 hours. The D-gal is sourced from Sigma.
2) Preparation of LPS Inducer: weigh 6.9 mg of LPS and add 6.9 mL of NS. Dissolve by vortexing to obtain the first solution with a concentration of 1 mg/mL. Divide the first solution into 100 µL tubes and store at-80 ° C. On the day of the experiment, take one tube and add 0.9 mL of NS to prepare the second solution with a concentration of 100 µ g/mL. Take 0.4 mL of the second solution, add 9.6 mL of NS, and mix thoroughly to obtain the LPS inducer with a final concentration of 4 µg/mL. Store the LPS inducer at 2-8° C and use it within 2 hours. The LPS was sourced from Shanghai Maokang Biotechnology Co., Ltd.
3) Modeling method: After acclimatization for 2 days, 24 mice were randomly selected and weighed. A single intraperitoneal injection of D-gal inducer (500 mg/kg) at 10 ml/kg was administered, followed by a single intraperitoneal injection of LPS inducer (40 µg/kg) at 10 ml/kg 3 hours later to establish a hepatic failure mouse model. Mouse strain: C57BL/6N; Mouse grade: SPF; Mouse sex: male; Mouse age: 8 weeks; Number of mice: 24; Source of mice: Zhejiang Weitong Lihua Laboratory Animal Technology Co., Ltd.; License number for mice: SCXK (Beijing) 2021-0006; Certificate number for mice: 1100112301103117632.
4) Drug Preparation: Remove several cell lines B42 from the liquid nitrogen tank and transfer them onto dry ice, immediately immerse the cell lines b42 in a 37° C water bath, by following gently shaking until the liquid completely melts, and ensuring uniform mixing. Note that the hose connector should not be submerged in the water bath, use a pipette to transfer all the liquid into a 50 ml sterile centrifuge tube, and entrifuge at 200g for 5 minutes. After centrifugation, discard the supernatant and dilute the cell lines b42 with normal saline to a concentration of 5 × 10^6 cells/ml. Before use, filter the solution through a 40 µm cell filter to obtain the cell suspension B42.
5) Animal grouping:
The hepatic failure mouse model was randomly divided into a control group and an experimental group, with 12 mice in each group. The control group received 0.2ml of normal saline via tail vein injection 30 minutes prior to the injection of the LPS inducer, as specified in Table 3. The experimental group received 0.2ml of an injection containing cell line B42 via tail vein injection 30 minutes prior to the injection of the LPS inducer, as specified in Table 3.

**Table 3 Animal Grouping Table**

| **Group** | **Number of animals** | **Sex** | **Medicine** | **Dosage** | **Route of medication** | **Freque ncy** |
|---|---|---|---|---|---|---|
| Compare group | 12 | Male | Normal saline | 0.2ml per unit | Caudal vein | Single |
| Experimen tal group | 12 | Male | Injection containing cell line B42 | 0.2ml per unit | Caudal vein | Single |

6) Injection of the test substance
The procedure for intravenous injection is as follows:
a. The mouse acute liver failure model was loaded into the fixator;
b. The tail of the mouse acute liver failure model was straightened and secured, then wiped with alcohol for disinfection;
c. The cells were uniformly dispensed, and 0.2ml of the injection containing cell line b42 was aspirated with a syringe;
d. The syringe needle was inserted into the tail vein of the mouse acute liver failure model, and the injection containing cell line b42 was injected, with a push-pull time of approximately 1 minute;
e. After the push-pull was completed, the syringe was withdrawn, and a dry cotton ball was applied to the puncture site to stop the bleeding; and
f. The mouse acute liver failure model was returned to the cage for rearing.

7) Clinical Observation
After injection of the solution containing cell line B42 in the liver failure mouse models of control group and experimental group, no drug-related adverse reactions were observed in the liver failure mouse models of control group, while the liver failure mouse models of experimental group mainly showed symptoms of reduced activity, lethargy and low body temperature.
8) Survival rate

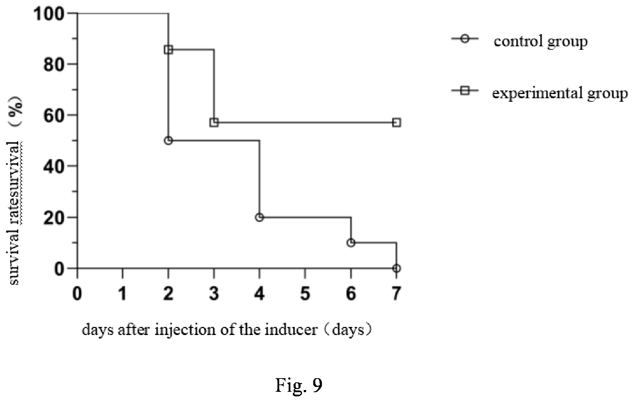

Fig. 9 is a comparative schematic diagram of the survival rates in the experimental group and control group of mice with acute liver failure models in an embodiment of the present invention application.

In the acute liver failure model of mice in the experimental group, the survival status of the mice was observed and the time to death was recorded after injection of D-gal inducer (500 mg/kg) combined with LPS inducer (40 µg/kg). Survival curves were plotted using GraphPad Prism 8. As shown in Fig. 9, compared with the control group, the survival rate of the acute liver failure model in the experimental group was 60%, while that in the control group was 0%. The experimental group exhibited a significant difference, indicating that hepatic precursor cells have a significant therapeutic effect on the acute liver failure model induced by d-gal inducer (500 mg/kg) combined with LPS inducer (40 µg/kg).

While the embodiments of the present invention application have been described in detail above, it is clear to those skilled in the art that various modifications and variations can be made to these embodiments. However, such modifications and variations shall be understood to fall within the scope and spirit of the present invention application as set forth in the claims. Furthermore, the present invention application described herein may have other embodiments and can be implemented in multiple ways.

## Claims

1. A method for establishing a hepatic precursor cell line, comprising following steps:
S0: preparing hepatic precursor cells, miscible liquids, and a reprogramming culture medium, wherein the miscible liquids comprises a serum-free medium, polyethyleneimine, and a CLP plasmid;
S1: transfecting the hepatic precursor cells with the miscible liquids to obtain transduced hepatic precursor cells; and
S2: passing the transduced hepatic precursor cells into the reprogramming culture medium for subculture to obtain immortalized hepatic precursor cells.

2. The method for establishing the hepatic precursor cell line according to claim 1, wherein the volume ratio of the serum-free medium to the polyethyleneimine is (500-1000):1.

3. The method for establishing the hepatic precursor cell line according to claim 1, wherein the final concentration of the CLP plasmid is (1-5) µg/ µL.

4. The method for establishing the hepatic precursor cell line according to claim 1, wherein the step of providing hepatic precursor cells comprises: culturing hepatocytes in the reprogramming culture medium for expansion, and then replacing the reprogramming culture medium with a serum-reduced medium to continue the culture and obtain hepatic precursor cells.

5. The method for establishing the hepatic precursor cell line according to claim 1, wherein the reprogramming culture medium comprises a DMEM/F12 basal medium and, in terms of the content relative to the DMEM/F12 basal medium: 15-25 ng/mL of hepatocyte growth factor (HGF), 15-25 ng/mL of epidermal growth factor (EGF), 5-15 µM of ROCK kinase inhibitor Y27632,1-5 µM of Wnt signaling pathway agonist CHIR99021,1-5 µM of TGF-β signaling inhibitor A8301,1-5 µM of sphingosine-1-phosphate (S1P), and 5 µ M of indole-3-acetic acid (LPA).

6. The method for establishing the hepatic precursor cell line according to claim 5, wherein the reprogramming culture medium further comprises N2 nutritional supplement, B27 nutritional supplement, sodium pyruvate, and ascorbic acid.

7. The method for establishing the hepatic precursor cell line according to claim 1, wherein the reprogramming culture medium comprises a DMEM/F12 basal medium and, in terms of the content relative to the DMEM/F12 basal medium: 1-10% N2 nutrient supplement, 1-10% B27 nutrient supplement, 1 mM sodium pyruvate, 5-15 µg/mL ascorbic acid, 15-25 ng/mL hepatocyte growth factor (HGF), 15-25 ng/mL epidermal growth factor (EGF), 5-15 µM ROCK kinase inhibitor Y27632,1-5 µM WNT signaling agonist CHIR99021,1-5 µ M TGF- β signaling inhibitor A8301,1-5 µ M S1P (sphingosine-1-phosphate), and 5 µM indole-3-acetic acid (LPA).

8. A hepatic precursor cell prepared by the method for establishing the hepatic precursor cell line as claimed in any one of claims 1-7.

9. The hepatic precursor cell according to claim 8, wherein the hepatic precursor cell negatively expresses CD34, CD45, and HLA-DRPQ, and positively expresses CD24 and CD326.

10. An application of the hepatic precursor cell as claimed in any one of claims 8-9, which is used in the preparation of a drug for the treatment of acute liver failure.
